# EUROPEAN PATENT APPLICATION

(11) **EP 4 020 134 A1**
(43) Date of publication of application: **29.06.2022**
(21) Application number: 21151959.0
(22) Date of filing: 16.01.2021
(51) Int. Cl.: G06F 3/01, A61B 5/16, F24F 11/00, G06N 3/08

(54) **METHOD FOR CONTROLLING A CUSTOMIZED MICROCLIMATE IN A BUILDING AND REALIZATION SYSTEM THEREOF**

(30) Priority: 22.12.2020 LT 2020049
(71) Applicant: Vilniaus Gedimino technikos universitetas, 10223 Vilnius (LT)
(72) Inventor: KAKLAUSKAS, Arturas, 08402 Vilnius (LT); BUCINSKAS, Vytautas, 10231 Vilnius (LT); DZEDZICKIS, Andrius, 14188 Vilnius (LT); URBAITE, Ieva, 03138 Vilnius (LT)
(74) Representative: Draugeliene, Virgina Adolfina

(57) **Abstract**

The present invention relates to indoor climate control method for buildings and a system embodying the control method that creates a certain effect and analyses levels of a person's productivity and valence taking into account the current climate in a fixed or mobile workplace. For digital assessment of the occupant's productivity and valence taking into account data on indoor climate data and the occupant's emotional, affective and physiological states stored in an affective computer memory, neuro decision matrices are generated in real time and then used as a basis to generate multiple indoor climate alternatives and calculate their utility degrees, priority and values; then, based on the data from such neuro decision matrices, the affective computer generates neuro-correlation matrices to show the interdependencies that link the occupant's productivity and valence to indoor climate data, and on the basis of this data the affective computer then determines any necessary climate adjustment coefficients as control data.

## Description

### Technical field to which invention relates

The present invention relates to the indoor climate control method for buildings and a system for realization the control method that creates a certain effect and analyses levels of a person's productivity and valence taking into account the current climate in a fixed or mobile workplace. Such systems make it possible to analyse and improve the overall labour efficiency, and in particular the maintenance and extension of the period of valuable human productivity, and allow making changes to indoor climate data to adjust them to an employee's short- and medium-term responses. This system is based on long-term research and makes use of the advances in artificial intelligence (Al) systems.

### Indication of the background art

In recent years, various emotional and affective indicators have been studied, including the net emotional value, the service encounter emotional value, the hedonic value and the perceived value, to establish a personal response assessment criterion and express it numerically.

The Net Emotional Value (NEV) is one such criterion, calculated by subtracting estimated negative emotions from estimated positive emotions. To calculate NEV, average negative emotion (dissatisfied, frustrated, disappointed, irritated, tense, unhappy, ignored, rushed) intensity are subtracted from average positive emotion (happy, satisfied, confident, valued, offered attentive service, safe, focused, forgiving, stimulated, exploring, interested, vigorous) intensity. The higher the criterion value, the better the person feels.

Over time new biometric methods, based on instrument-based data (e.g. heart rate, heart rate variability, breathing rate, galvanic skin response, eye tracking, pupil dilation, EEG, ECG) monitoring, were added to the pool of human mood and productivity measurement techniques. Biometrics also emerged, a new specialised field of research that makes use of various biometric tools (e.g. functional magnetic resonance imaging (fMRI), electroencephalography, facial expressions, the tone of voice, heart rate). They aid in recognising an employee's responses to indoor-climate-induced stimuli classified in line with the SIS International Market Research methodology.

Several technical solutions for personalised indoor climate control in buildings are currently known. They present technical solutions for physiological parameters measurement, define the devices to determine data that describe human emotions, but do not present directly the proposed indoor climate control method and the devices for its embodiment.

One field of climate control application, a car climate control system, is described in the US patent US9677894B2 that presents a case of using human biometric data to adjust cabin climate control data inside a car. This climate system has its data controlled automatically based on pre-set climate data determined after biometric recognition identifies a driver or a passenger. The system is adaptive in the way it selects pre-set human profiles, but does not take into account real-time parameters changes. On the other hand, the setting of a profile's climate data is only the user's external assessment with the human body's response ignored.

The US patent US8478447B2 describes a climate control system consisting of multiple climate adjustment microdevices controlled from a single computer-based control centre. A system of this type makes it possible to work on climate adjustments in case of contradictory temperature and humidity demands for various zones in living or work spaces. Such systems do not offer direct biometric control.

The US patent application US20110272131A1 describes a system for controlling the climate in a car cabin that controls air temperature and airflow velocity and either turns them up or down. The control data are determined by a special handheld sensor. The temperature of and the amount of sweating on the hand determines the values of climate data and sensor data are sent to the climate control system by wireless means. The system's quick operation has not been described; the data it determines remain fairly static and do not respond to the sensations experienced by a person or the person's tiredness.

The US patent application US20140310105A1 describes a system of wearable sensors and a computer system for processing their signals to describe an individual's state and control various devices on that basis, including a personalised climate control system. This invention makes it possible to base device control on direct sensor input, but its technical solution does not take into account emotional states.

A similar solution is presented in the US patent application US20140222735A1 where a computer is used to transcript mood and assess sensor readings. A computer-system-implemented method analyse the individual's mood is at least one of anger, sadness, happiness, enjoyment, neutral, ambivalence, frustration, and surprise, and further comprise providing targeted content based on the individual's determined mood. The solution also produces a control signal based on the signal received from a wearable sensor, but the mood are not decoded or analysed.

An advanced climate control system is presented in the US patent US9020647B2. The system uses industry comfort climate setting data stored in a permanently connected database. Each employee sets a comfortable climate level, then individuals are recognised by their biometric data and climate is adjusted for each individual. The system is not interactive in terms of personal comfort; it responds by recognising individuals in various spaces of a building.

A technical solution to determine the physical performance of an individual and express it as an objective criterion value is described in the US patent US9478149B2. This patent focuses on instant assessment of the physical state of an individual, but is hardly designed to determine intellectual productivity. This invention reflects biometric data of individuals and their processing to determine characteristics, but does not offer a technical solution appropriate for climate parameters control.

The patent application US20190208488A1 describes an affective computing server (ACS) incorporated into a mobile wireless network management infrastructure. ACS, over a mobile wireless network, maintains optimal personalised comfort status of a user of a wearable sensors network (e.g. biomarker sensors, user identification, current user location). ACS, over mobile wireless network technology, receives data on the user's comfort status profile. Based on the received data on the user's comfort status profile, ACS changes climate data (temperature, humidity, air quality) in a fixed or mobile space. Tuned climate spaces, fixed or mobile, are important in the context of smart cities, homes and residential spaces, where the proposed system and the method of operation personalise an environment meeting personal needs of users to maximise comfort/satisfaction of multiple users. A wide range of indoor environments within a managed space leads to a varied distribution of ambient indoor temperatures. The proposed system and method adapt the climate in a fixed or mobile environment to suit unique physiological, psychological and comfort profiles of specific occupants. This is done by taking into account the dynamic nature of indoor environment conditions to meet instantaneous user comfort and needs. Based on the user biomarker data detected by the wearable sensors and the pre-set preferences of the specific user(s), the proposed system makes dynamic adjustments of indoor climate data to suit personal user comfort profiles, together with user wellness monitoring.

The known methods and systems help with personalised climate control in fixed or mobile environments, but cannot be applied to determine various related values (utilitarian, hedonic, user perceived, emotional and other values) that would offer a more accurate explanation of user comfort status, responses, feelings and preferences. The aim is, therefore, to have an improved method and system available that would give stakeholders smart climate control for a fixed or mobile environment. In addition, this method and system do not offer repetitive, average, strong and very strong statistical relationships between variables that would make it possible to get an accurate and objective view of a personalised climate control process in a fixed or mobile environment. They do not, for instance, determine real-time neuro-correlations (statistical) relationships between the ambient indoor climate and user biomarker data with the help of probability theory or statistical methods. The know method, therefore, does not make it possible to learn more about the efficiency of indoor climate changes in an environment at each stage of the process to help determine the advantages and disadvantages of climate changes in such environment.

Due to the aforementioned attributes, the known indoor climate control methods and devices for buildings are not accurate or reliable and have a narrow field of application.

### Technical problem to be solved

The invention aims to make the personalised indoor climate control method for buildings and the method's embodiment system more accurate and reliable and expand their scope of application by creating a method for indoor climate control in fixed or mobile workplaces taking into account a person's state and a system to embody the method that makes it possible to determine how a person feels and apply relevant indoor climate data to achieve the highest productivity and positive valence of the said person or establish the person's rest break schedule.

### Disclosure of the invention

In order to solve the technical problem according to a proposed invention is that in a method for controlling a customized microclimate in a building, wherein indoor climate is controlled depending on occupant's emotional, affective and biometric data reflecting the occupant's state, comprising-:
- real-time recording of indoor climate data to a computer memory,
- real-time monitoring and decoding of the occupant's emotional, affective and physiological state by a set of sensors,
- transmitting of the data about the occupant's emotional, affective and physiological state decoded by the set of sensors to a biometric information collection and conversion device,
- recording of the data collected and converted in the biometric information conversion device (3) to the computer memory, from which the data is transmitted to a central processing unit, which performs numerical evaluation the occupant productivity and valence in order to form control parameters of ambient indoor climate and transmits the control parameters to a climate microcontroller (5), the numerical evaluation of the occupant's productivity and valence performed by the central processing unit of the computer comprises the following steps:
   - forming in real time neuro-decision matrices, by performing an evaluation of the indoor climate data stored in the computer memory according to the levels of the occupant's emotional, affective and physiological states,
   - forming indoor climate alternative variants on the basis of the data of neuro-decision matrices and computing their utility degrees, priority and values, indicating the values and significance of the levels of the occupant's states, and storing their in the computer memory,
   - forming neuro-correlation matrices of productivity and valence by the central processing unit of the computer on the base of the data of the neuro- decision matrices stored in the computer memory, where the formed neuro-correlation matrices (10) show the interrelationships between the occupant's productivity and valence and indoor climate data, and records the data to an indoor climate model stored in the memory,
   - transmitting the data stored in the indoor climate model to the central processing unit, which determines any necessary indoor climate adjustment coefficients in the form of control data, the coefficients are recorded as updates in the indoor climate model stored in the memory and then sent via a biometric network adapter as control commands to the microcontroller, which controls indoor climate control devices in real time.

System for controlling a customized microclimate in a building, wherein indoor climate is controlled taking into account an occupant's biometric data reflecting the occupant's emotional, affective and physiological states, comprising:
a set of sensors, associated with an occupant, a device for collection biometric information from the set of sensors and its conversion to digital data, which are recorded to a computer memory, from which the data are transmitted to a central processing unit, which performs numerical evaluation the occupant productivity and valence in order to form control parameters of ambient indoor climate and transfers the control parameters to a climate microcontroller, wherein performing numerical evaluation of the occupant productivity and valence to form control parameters of ambient indoor climate
   the central processing unit of the computer is configured in order to form in real time neuro-decision matrices by performing an evaluation of the indoor climate data stored in the computer memory according to the levels of the occupant's emotional, affective and physiological states on the base of which indoor climate alternative variants are formed and utility degrees, priority and values of the alternative variants are computed, indicating the values and significance of the levels of the occupant's states, and stores their in the computer memory, and
the central processing unit is configured in order to form neuro-correlation matrices on the base of the data of the neuro-decision matrices stored in the computer memory, that show the dependencies linking the occupant's productivity and valence to indoor climate data, neuro-correlation matrices data are transmitted and recorded to an indoor climate model stored in the memory, from which the stored data are sent to the central processing unit, which determines any necessary climate adjustment coefficients in the form of control parameters and the coefficients are then recorded as updates to the indoor climate model stored in the memory and transmitted via a biometric network adapter as control commands to the microcontroller, which manages indoor climate control devices in real time.

Data received from the system's devices are processed and stored in cloud computing resources.

### Advantages of the invention

The personalised indoor climate control method for buildings and its embodiment system assess an occupant's emotional and affective states by processing data in an affective computer, and assess the person's physiological state and the level of performance that determine optimal climate data for a fixed or mobile workplace. The data are determined dynamically to produce small indoor climate adjustments at set intervals taking into account the occupant's productivity and valence in real time to find optimal climate parameters for the workplace environment for that specific time period. The system is universal and independent of the climate zone, but it depends on the type of an indoor space. It enables preventive analysis of emotional, affective and physiological reactions of employees and can respond to productivity and valence changes that can affect the rhythm of work assignments or the schedule of rest breaks if the work planned cannot be done or its performance can be riddled with errors. The proposed method and the devices required for its embodiment make it possible, with the help of research-based methods, to determine optimal indoor climate data that match the occupant's period of productivity (e.g. the Yerkes-Dodson Law) and positive valence.

The proposed method and its embodiment system use special data processing procedure with neuro decision matrices, which serve as a basis to generate indoor climate alternatives and calculate their utility degrees, priority and values. Then neuro-correlation matrices are used to look for the relationships between the data and determine the strength of those relationships; such neuro-correlation matrices show the interdependencies linking the occupant's productivity and valence to indoor climate data and are used as a basis in the selection of climate control data. In addition, these matrices ensure maximum reliability of data relationships that match the actual effect of indoor climate conditions on the employee, thus the proposed method and embodiment system offer high control accuracy, reliability and can be widely applied in all areas where control of indoor climate based on an occupant's state is required in a fixed or mobile (e.g. a car) workplace.

With the help of multiple criteria decision methods (such as INVAR) that use real-time neuro decision matrices, real-time utilitarian, hedonic, user perceived, emotional and other values for indoor spaces are calculated. These values must fall within pre-set minimum (user needs the least satisfied, but very low operating costs of indoor spaces) and maximum (user needs the most satisfied, but very high operating costs of indoor spaces) boundaries. If the value falls within the minimum and maximum boundaries, the indoor climate remains unchanged, but when the data fall outside the boundaries, the climate is adjusted again taking into account the pre-set statistical productivity and valence neuro-correlation matrices. Neuro decision matrices are used for that specific purpose.

One of the most important aspects that make this patent innovative is that utilitarian, hedonic, user perceived, emotional and other values of indoor spaces are determined in real time. A neuro decision matrix includes columns that describe specific indoor spaces n and rows m that show an employee's emotional state (e.g. happy, sad, angry, scared, disgusted), affective state (e.g. bored, interested, confused), valence, arousal and physiological state (e.g. face temperature, heart rate, breathing rate, galvanic skin response, eye tracking, pupil dilation, EEG, ECG) changing in real time and indoor climate data that describe indoor spaces and their occupants in detail. Arousal is determined based on physiological user data, facial expression recognition software. The neuro decision matrix also includes measuring units, criteria weights, and a minimising/maximising criterion. This facilitates real-time multiple criteria analysis of indoor climate and the emotional state, affective state, valance, arousal and physiological state of users to determine the values for indoor spaces, and makes the physical purpose of the calculations clearer. Real-time multiple criteria analysis of indoor climate and the emotional state, affective state, valence, arousal and physiological state of users usually means that large amounts of information must be processed; it is, therefore, rational to use matrices for such processing. In this case, indoor climates, occupants and the data that describe them are grouped in a certain way, i.e. a neuro decision matrix is generated. When the criteria intensity and weights have been determined (the weights are pre-set by users and experts), multiple criteria analysis methods (such as INVAR) are applied to determine the utility degree, priority and values (utilitarian, hedonic, user perceived, emotional and other values) for indoor spaces in real time.

Real-time neuro-correlations (correlation coefficients) are also determined among multiple indoor climate data and multiple emotional state, affective state, valence, arousal and physiological state data of users. Probability theory or statistical methods are applied to determine these real-time statistical relationships between indoor climate and the emotional state, affective state, valence, arousal and physiological state variables of users. Several different correlation coefficients can be used, such as Pearson's correlation coefficient, Spearman's rank-order correlation or others. Indoor climate control devices can measure variables such as indoor humidity, temperature, lighting and air pollution, which have strong correlations with the emotional state, affective state, valence, arousal and physiological state of users determined by a set of sensors and climate control devices. This means that the variables of the emotional state, affective state, valence, arousal and physiological state of users make a direct impact on the decision to set personalised indoor climate.

### Brief description of the drawings

Fig. 1 shows illustrative block scheme of system for realization of method for controlling a customized microclimate in a building
Fig. 2 shows a workflow process of the realization system presented in Fig. 1.
Fig. 3 shows the interdependency between arousal and productivity.
Fig. 4 shows the data processing procedure with neuro decision matrices and neuro-correlation matrices generated.

### One of the examples of the embodiment of the invention

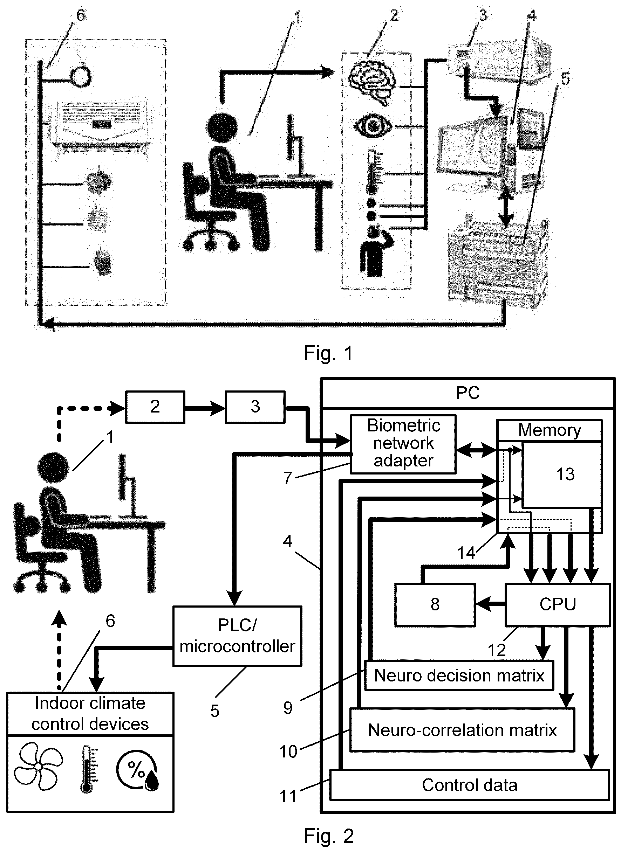

The embodiment system of personalised indoor climate control for buildings shown in Fig. 1 is designed to analyse the emotional, affective and physiological data of the occupant 1 as part of the climate control process. The indoor climate control devices 6 of a specific indoor space of a specific building is used to determine and control the indoor air quality (e.g. temperature, humidity, lighting, air pollution), and a set of sensors 2 that measures the occupant's emotional, affective and physiological data spanning multiple physiological reactions is used to determine the occupant's emotional state (e.g. happy, sad, angry, scared, disgusted), affective state (e.g. bored, interested, confused), valence, arousal and physiological state (e.g. face temperature, heart rate, breathing rate, galvanic skin response, eye tracking, pupil dilation, EEG, ECG). The set includes sensors 2 for electroencephalography (EEG), pupil response, heart rate, breathing rate, body temperature and its variation, perspiration and many other known physiological responses. These sensors 2 by applying a data bus transfers data directly to a biometric information conversion device 3 connected to an affective computer (PC) 4 which normalises (groups by weight and removes repetitions) and analyses the data, and generates control commands for the climate control PLC/microcontroller 5. Biometric information conversion device 3 is used to convert the user biometric image, which is transmitted and saved to the affective computer 4. The bus connection between the biometric information conversion device 3 and the affective computer 4 can be either standard wireless link (e.g. Wi-Fi, 5G, 4G mobile wireless) or specialised with an additional data channel in case large amounts of data need processing. The PLC/microcontroller 5 receives data from the affective computer 4 and manages indoor climate control devices (e.g. HVAC equipment) 6 that maintains the indoor air quality (e.g. temperature, humidity, lighting, air pollution) data.

Fig. 2 shows a flowchart of the embodiment system of personalised indoor climate control for buildings presented in Fig. 1. The system's operation starts with the set of sensors 2 that measure the emotional, affective and physiological data of the occupant 1. These measurements are then sent to the biometric information conversion device 3, which then transfers the measurements to the memory 14 of the main affective computer (PC) 4 via a biometric network adapter 7. The data from the set of sensors 2 are added to the indoor climate model 13 stored in the memory and sent to a central processing unit (CPU) 12 for determining the level of emotions 8. The level of emotions, expressed in the form of the level of valence and arousal in the circumplex model of affect, is stored in the affective computer memory. Indoor climate data are also collected in real time to the affective computer memory (14). In Step 2 of the data processing, the emotion level data are again transferred to the CPU 12, which makes an assessment of the indoor climate data stored in the affective computer memory (14) against the levels of the occupant's emotional, affective and physiological states and generates in real time neuro decision matrices (9), which are then used as a basis to generate indoor climate alternatives and calculate their utility degrees, priority and values. The data from the neuro decision matrices 9 are written to the memory 14. In Step 3, the data from the neuro decision matrices 9 are sent to the CPU 12 and the CPU generates neuro-correlation matrices 10 that show the interdependencies between the occupant's productivity and valence and indoor climate data; these matrices are used as a basis to select climate control data. These data are added to the indoor climate model 13 stored in the memory 14; the model describes the relationships between the level of human emotional, affective and physiological states and indoor climate. In Step 4 of the data processing, the data from the indoor climate model stored in the memory are sent to the CPU 12, which determines any necessary indoor climate adjustment coefficients in the form of control data 11; these coefficients are added as updates to the indoor climate model 13 stored in the memory 14 and transferred as control commands via the biometric network adapter to the PLC/microcontroller 5, which controls the indoor climate control devices 6. The present personalised indoor climate control method and its embodiment system are designed both for fixed and mobile (e.g. a car) environments.

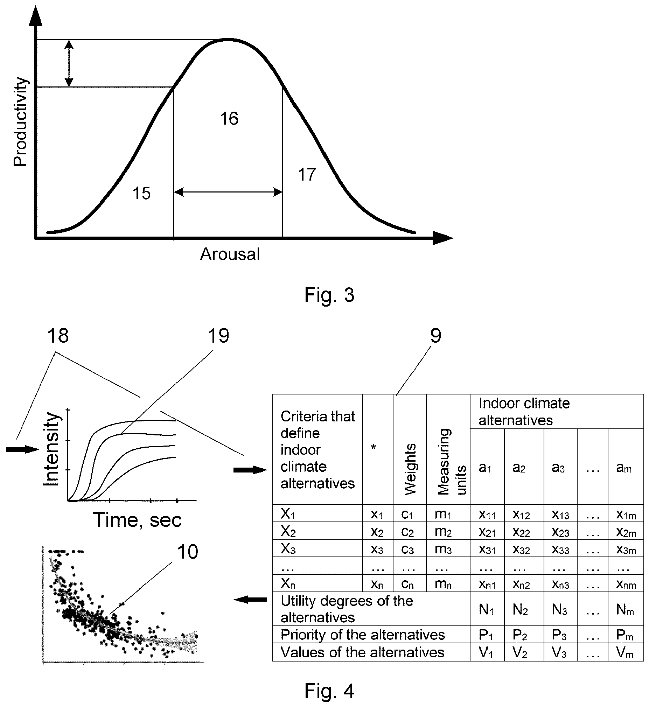

Decision making for the embodiment system of personalised indoor climate control for buildings takes into account the dependency (shown in Fig. 3; e.g. the Yerkes-Dodson Law) between arousal (e.g. arousal is determined based on physiological user data, facial expression recognition software) and productivity divided into three segments: the arousal level within Segment 15 means motivation, focus, interest, stress and anxiety are going up, work feels more and more comfortable; the arousal level within Segment 16 means a rational level of stress and arousal has been achieved with optimal productivity ensured; and the arousal level within Segment 17 means a drop in work efficiency due to intense stress and arousal. The embodiment system for personalised indoor climate control is designed to keep the arousal level in Segment 16 at all times (Fig. 3).

Fig. 4 shows the flow of data processing, where the signals received from the set of sensors 2 are sent as a data stream 18 delivered in the form of a time series 19. In the next data processing and analysis step, neuro decision matrices 9 are generated at a pre-set time step and then used as a basis to generate indoor climate alternatives and calculate their utility degrees, priority and values. Then, based on the neuro decision matrices 9 stored in the memory 14, the CPU 12 generates neuro-correlation matrices 10 that show dependencies between the occupant's productivity and valence and indoor climate data and then, based on these matrices, the selection of climate control data is done.

To implement the structural diagram and the flowcharts shown in Figures 1-4, a PC or cloud computing resources are used. The embodiment system is connected to a general affective computer network or is part of such network. Data from biometric sensors (emotional, affective and physiological states) 2 are sent, via wireless link (e.g. Wi-Fi, 5G, 4G mobile wireless), to the affective computer 4 for further processing. The indoor climate adjustment level generated by the affective computer 4 is sent to the PLC/microcontroller 5 with power switching/control circuits that control the indoor climate maintenance devices such as HVAC (heating, ventilation, and air conditioning).

## Claims

1. Method for controlling a customized microclimate in a building, wherein indoor climate is controlled depending on occupant's emotional, affective and biometric data reflecting the occupant's state, comprising-:
- real-time recording of indoor climate data to a computer memory (14),
- real-time monitoring and decoding of the occupant's emotional, affective and physiological state by a set of sensors (2),
- transmitting of the data about the occupant's emotional, affective and physiological state decoded by the set of sensors (2) to a biometric information collection and conversion device (3),
- recording of the data collected and converted in the biometric information conversion device (3) to the computer memory (14), from which the data is transmitted to a central processing unit (12), which performs numerical evaluation the occupant productivity and valence in order to form control parameters of ambient indoor climate and transmits the control parameters to a climate microcontroller (5) **characterized in that** the numerical evaluation of the occupant's productivity and valence performed by the central processing unit (12) of the computer (4) comprises the following steps:
- forming in real time neuro-decision matrices (9), by performing an evaluation of the indoor climate data stored in the computer memory (14) according to the levels of the occupant's emotional, affective and physiological states,
- forming indoor climate alternative variants on the basis of the data of neuro-decision matrices (9) and computing their utility degrees, priority and values, indicating the values and significance of the levels of the occupant's states, and storing their in the computer (4) memory,
- forming neuro-correlation matrices (10) of productivity and valence by the central processing unit (12) of the computer (4) on the base of the data of the neuro-decision matrices (9) stored in the computer memory (14), where the formed neuro-correlation matrices (10) show the interrelationships between the occupant's productivity and valence and indoor climate data, and records the data to an indoor climate model (13) stored in the memory 14,
- transmitting the data stored in the indoor climate model (13) to the central processing unit (12), which determines any necessary indoor climate adjustment coefficients in the form of control data (11), the coefficients are recorded as updates in the indoor climate model (13) stored in the memory (14) and then sent via a biometric network adapter (7) as control commands to the microcontroller (5), which controls indoor climate control devices (6) in real time.

2. System for controlling a customized microclimate in a building, wherein indoor climate is controlled taking into account an occupant's (1) biometric data reflecting the occupant's emotional, affective and physiological states, comprising:
a set of sensors (2), associated with an occupant (1),
a device (3) for collection biometric information from the set of sensors (2) and its conversion to digital data, which are recorded to a computer (4) memory (14), from which the data are transmitted to a central processing unit (12), which performs numerical evaluation the occupant productivity and valence in order to form control parameters of ambient indoor climate and transfers the control parameters to a climate microcontroller (5) **characterized in that** for performing numerical evaluation of the occupant productivity and valence to form control parameters of ambient indoor climate
the central processing unit (12) of the computer (4) is configured in order to form in real time neuro-decision matrices (9) by performing an evaluation of the indoor climate data stored in the computer memory (14) according to the levels of the occupant's emotional, affective and physiological states on the base of which indoor climate alternative variants are formed and utility degrees, priority and values of the alternative variants are computed, indicating the values and significance of the levels of the occupant's states, and stores their in the computer memory (14), and
the central processing unit (12) is configured in order to form neuro-correlation matrices (10)) on the base of the data of the neuro-decision matrices (9) stored in the computer memory (14), that show the dependencies linking the occupant's productivity and valence to indoor climate data, neuro-correlation matrices (10) data are transmitted and recorded to an indoor climate model (13) stored in the memory (14), from which the stored data are sent to the central processing unit (12), which determines any necessary climate adjustment coefficients in the form of control parameters (11) and the coefficients are then recorded as updates to the indoor climate model (13) stored in the memory (14) and transmitted via a biometric network adapter (7) as control commands to the microcontroller (5), which manages indoor climate control devices (6) in real time.

3. The system of claim 2, **charecterized in** that the data received from the system's devices are processed and stored in cloud computing resources.
